# EUROPEAN PATENT SPECIFICATION

(11) **EP 1 522 290 B1**
(45) Date of publication and mention of the grant of the patent: **24.05.2006**
(21) Application number: 04104863.8
(22) Date of filing: 04.10.2004
(51) Int. Cl.: A61F 9/007

(54) **Surgical wide angle illuminator**
Chirurgisches Weitwinkelbeleuchtungsgerät
Instrument chirurgical d'éclairement à grand angle

(30) Priority: 08.10.2003 US 509479 P; 30.10.2003 US 697350
(43) Date of publication of application: 13.04.2005
(73) Proprietor: Alcon, Inc., 6331 Hünenberg (CH)
(72) Inventor: Hickingbotham, Dyson, PA 19567, Stouchsburg (US)
(74) Representative: Hanna, Peter William Derek

(56) References cited:
- US-A- 4 222 375
- US-A- 5 514 125
- US-A- 5 782 825
- US-A- 5 784 508
- US-B1- 6 428 553

## Description

### TECHNICAL FIELD OF THE INVENTION

The present invention relates generally to surgical instrumentation. In particular, the present invention relates to surgical instruments for illuminating an area during eye surgery. Even more particularly, the present invention relates to a variable intensity, small gauge, wide-angle illuminator for illumination of a surgical field.

### BACKGROUND OF THE INVENTION

In ophthalmic surgery, and in particular in vitreo-retinal surgery, it is desirable to use a wide-angle surgical microscope system to view as large a portion of the retina as possible. Wide-angle objective lenses for such microscopic systems exist, but they require a wider illumination field than that provided by the cone of illumination of a typical fiber-optic probe. As a result, various technologies have been developed to increase the beam spreading of the relatively incoherent light provided by a fiber-optic illuminator. These known wide-angle illuminators can thus illuminate a larger portion of the retina as required by current wide-angle surgical microscope systems. Currently existing wide-angle illuminators, however, display several disadvantages.

One disadvantage exhibited by some prior art wide-angle illuminators for ophthalmic surgery is a matching of the light refracting index of the vitreous eye fluid to that of the light refracting surface of the lens of the illuminator that comes in contact with the vitreous eye fluid. Contact of the vitreous eye fluid with the light refracting surface of the light spreading lens of such prior art systems results in sub-optimal light refraction due to index switching caused by the vitreous eye fluid. U.S.-A- 5,624,438, (Turner) entitled "Retinal Wide-Angle Illuminator For Eye Surgery," provides a system for overcoming the effect of refractive index matching through the use of a high refractive-index step, mediated by the presence of an air-gap. The air-gap is presented between the distal end of an optical fiber and the light refracting surface of the illuminator lens. The light emanating from the optical wave guide (i.e., the optical fiber) will therefore undergo angular dispersion without any index switching that might be caused by contact with the vitreous eye fluid before it passes through the light refracting surface of the illuminator lens.

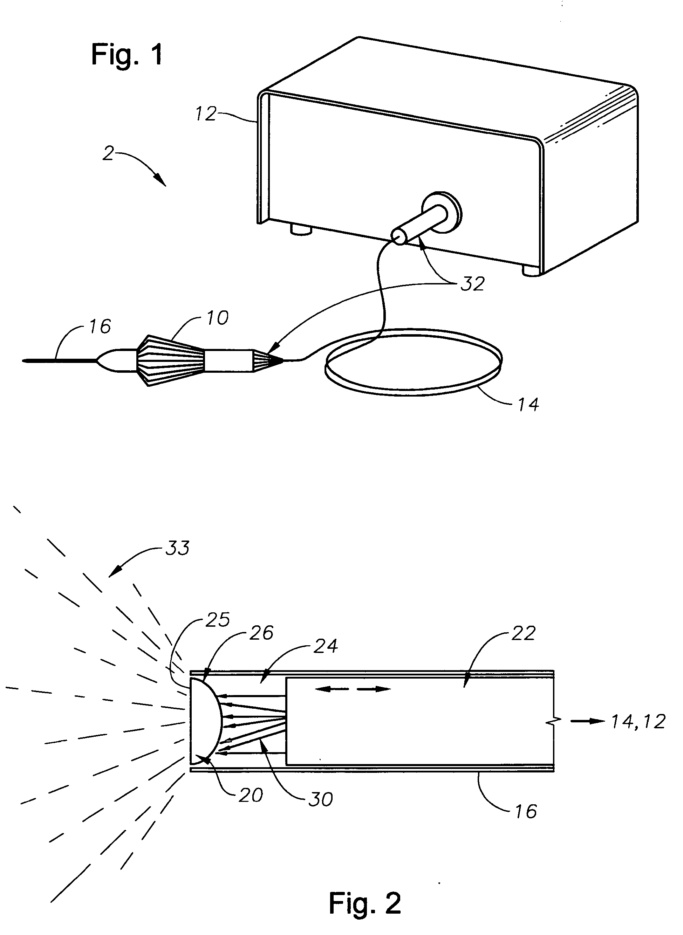

U.S.-A-5,784,508 (Turner), entitled "Ball, wide angle illuminator for eye surgery" describes a sapphire ball lens mounted at the distal end of an illuminator facing an optic fiber with a fixed air gap in between. Fig. 2 shows a hemispherical lens with a flat circular surface to the exterior which forms the basis for the preamble of claim 1.

Another disadvantage of currently available wide-angle illuminators is glare. Glare results when the source of the illumination is small and bright, and the user (e.g., an ophthalmic surgeon) has a direct line of sight to the small bright illumination source. Glare is unwanted stray radiation that provides no useful illumination, and either distracts an observer or obscures an object under observation. Glare can be corrected for in current wide-angle illuminators, but typically only by reducing the total illumination light flux, which reduces the amount of light available for observation by the surgeon. For example, the "bullet probe" manufactured by Alcon Laboratories, Inc., of Fort Worth, Texas, achieves wide-angle illumination by using a bullet-shaped fiber having a surface diffusive finish to scatter light emanating from the distal end of an optical fiber. To reduce glare, the bullet probe can use a geometric shield, which reduces the illumination angle by reducing the overall available light flux.

A further disadvantage of typical prior art wide-angle illuminators is that they do not provide for varying the illumination angle and/or the intensity of the light source to adjust illumination for different conditions within the surgical field. Further still, prior art wide-angle surgical illuminators are expensive to produce, a cost which is passed along to the surgeon and ultimately to the patient. As a result, prior art illuminators are typically not disposable and will require periodic maintenance and sterilization between surgical procedures.

Therefore, a need exists for a variable-intensity, wide-angle illuminator that can reduce or eliminate the problems of refractive-index matching, glare, adjustable illumination properties, cost, efficiency and other problems associated with prior art wide-angle illuminators.

### BRIEF SUMMARY OF THE INVENTION

The present invention provides embodiments of a variable-intensity, wide-angle surgical illuminator in accordance with claims which follow, with a view to substantially meeting these needs and others.

The invention is defined by the features of claim 1. Preferred embodiments are disclosed in the dependend claims.

The optical element can be a small-gauge, optical-grade diffusive sapphire element having a polished flat circular surface co-incident with the distal end of the cannula and a light refracting hemispherical surface facing the optical fiber. For example, the optical element can be sized for housing within a 19, 20 or 25 gauge cannula (e.g., about .75 mm to about .4 mm diameter optical element). Further, the cannula and the handpiece can be fabricated from biocompatible materials. The optical cable can comprise a first optical connector operably coupled to the light source and a second optical connector operably coupled to the handpiece (to optically couple the optical cable to the optical fiber housed within the handpiece and cannula). These connectors can be SMA optical fiber connectors. The optical element, optical fiber and optical cable (i.e., the optical fibers within the optical cable) should be of a compatible gauge so as to transmit the light beam from the light source to the surgical field. For example, all three elements could be of equal gauge.

To enable the advantages of this invention, the optical fiber is operably coupled to the handpiece to enable linear displacement of the optical fiber within the cannula. The sapphire optical element remains fixed relative to an open aperture of the cannula (i.e., co-incident with the open aperture edge). The handpiece can include a means, such as a push/pull mechanism, for adjusting the linear displacement of the optical fiber. Other adjusting means as known to those in the art can also be used. Adjusting the linear displacement of the optical fiber will change the distance between the optical element and the distal end of the optical fiber. When the optical fiber end is closest to the optical element, the light exiting the optical fiber end will be diffracted less before entering the optical element than when the optical fiber end is farther away from the optical element. Thus, by adjusting the linear displacement of the optical fiber (i.e., adjusting the distance between the optical fiber distal end and the optical element), the angle of illumination and the amount of illumination provided by the optical element from the light beam to illuminate the surgical field (e.g., the retina of an eye) can be adjusted by the surgeon. Embodiments of this invention can provide a range of illumination angles up to about 160 degrees (e.g., about 20 degrees to about 160 degrees).

Also disclosed is a method for wide-angle illumination of a surgical field using a variable-intensity, wide-angle illuminator in accordance with the teachings of this invention, and a surgical handpiece embodiment of the variable-intensity, wide-angle illuminator of the present invention for use in ophthalmic surgery. Embodiments of this invention can be implemented as a handpiece connected to a cannula, or other housing, including a fiber optic cable terminating in a diffusive optical element. Further, embodiments of this invention can be incorporated within a surgical machine or system for use in ophthalmic or other surgery. Other uses for a variable-intensity, wide-angle illuminator designed in accordance with the teachings of this invention will be known to those familiar with the art.

A more complete understanding of the present invention and the advantages thereof may be acquired by referring to the following description, taken in conjunction with the accompanying drawings, in which like reference numbers indicate like features and wherein:
FIGURE 1 is a simplified diagram of one embodiment of a system for variable, wide-angle illumination in accordance with the teachings of this invention;
FIGURE 2 is a more detailed diagram of a stem housing an embodiment of a diffusive optical element for wide-angle illumination in accordance with the teachings of this invention;
FIGURE 3 is a diagram illustrating the use of an embodiment of a wide-angle illuminator of the present invention for ophthalmic surgery; and
FIGURE 4 is a diagram illustrating an embodiment of an adjusting means 40 in accordance with the present invention.

### DETAILED DESCRIPTION OF THE INVENTION

Preferred embodiments of the present invention are illustrated in the FIGURES, like numerals being used to refer to like and corresponding parts of the various drawings.

The various embodiments of the present invention provide for a small gauge (e.g., 19, 20, or 25 gauge) optical fiber based endo-illuminator device for use in surgical procedures, such as in vitreo-retinal/posterior segment surgery. Embodiments of this invention can comprise a handpiece, such as the Alcon-Grieshaber Revolution-DSP™ handpiece sold by Alcon Laboratories, Inc., Fort Worth, Texas, connected to a small gauge cannula (e.g., 19, 20, or 25 gauge). The inner dimension of the cannula can be used to house one, or a plurality of, optical fibers terminating at a diffusive optical element in accordance with the teachings of this invention. Embodiments of the wide-angle illuminator can be configured for use in the general field of ophthalmic surgery. However, it is contemplated and it will be realized by those skilled in the art that the scope of the present invention is not limited to ophthalmology, but may be applied generally to other areas of surgery where wide-angle and/or variable illumination may be required.

An embodiment of the variable-intensity, wide-angle illuminator of this invention can comprise a light diffusive optical element fabricated of optical-grade sapphire, and a stem and a handpiece fabricated from biocompatible polymeric materials, such that the invasive portion of the wide-angle illuminator is a disposable surgical item. Unlike the prior art, each embodiment of the variable-intensity, wide-angle illuminator of this invention can provide high optical transmission/high brightness with low optical losses. Embodiments of this invention fabricated from biocompatible polymeric materials can be integrated into a low cost, articulated handpiece mechanism, such that these embodiments can comprise an inexpensive disposable illuminator instrument.

FIGURE 1 is a simplified diagram of a surgical system 2 comprising a handpiece 10 for delivering a beam of relatively incoherent light from a light source 12 through cable 14 to a stem 16. Cable 14 can be any gauge fiber optic cable as known in the art, but is preferably a cable having 19, 20, or 25 gauge fiber. Further, cable 14 can comprise a single optical fiber or a plurality of optical fibers optically coupled to receive and transmit light from light source 12 to stem 16 through handpiece 10. Stem 16 is configured to house a diffusive optical element 20 at the distal end of stem 16, as is more clearly illustrated in FIGURE 2. Coupling system 32 can comprise an optical fiber connector at each end of cable 14 to optically couple light source 12 to an optical fiber within handpiece 10, as discussed more fully below.

FIGURE 2 is a magnified view of the distal end of stem 16. Stem 16 is shown housing fiber 22 and optical element 20. Optical element 20 is optically coupled to fiber 22, which is optically coupled to fiber optic cable 14. In some embodiments, fiber optic cable 14 can extend through the handpiece 10 and is optically coupled directly to optical element 20. For these embodiments, fiber 22 is not used. When implemented within handpiece 10, fiber 22 is of a gauge compatible with the gauge of fiber optic cable 14, such that it can receive and transmit light from fiber optic cable 14. Handpiece 10 can be any surgical handpiece as known in the art, such as the Revolution-DSP™ handpiece sold by Alcon Laboratories, Inc. of Fort Worth, Texas. Light source 12 can be a xenon light source, a halogen light source, or any other light source capable of delivering relatively incoherent light through a fiber optic cable. Stem 16 can be a small gauge cannula, preferably on the order of 19, 20, or 25 gauge, as known to those in the art. Stem 16 can be stainless steel or a suitable biocompatible polymer (e.g., PEEK, polyimide, etc.) as known to those in the art.

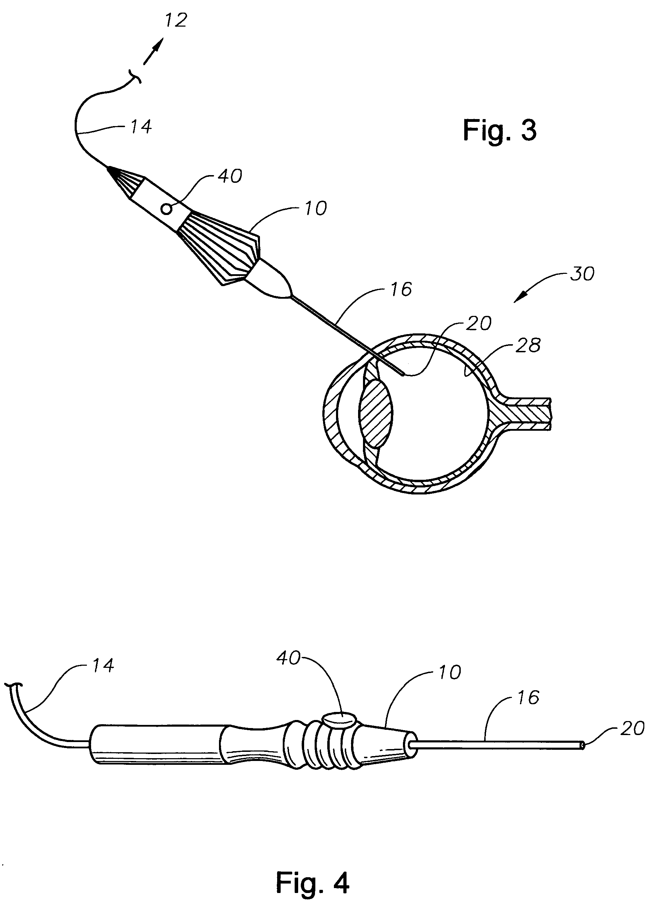

The fiber optic cable 14 or fiber 22 housed within the stem 16 is operably coupled to the handpiece 10, for example, via an adjusting means 40, as shown in FIGURE 4. Adjusting means 40 can comprise, for example, a simple push/pull mechanism as known to those in the art. Light source 12 can be optically coupled to handpiece 10 (i.e., to fiber 22) using, for example, standard SMA (Scale Manufacturers Association) optical fiber connectors at the proximal ends of fiber optic cable 14. This allows for the efficient coupling of light from the light source 12 through fiber optic cable 14 to the handpiece 10 and finally emanating from optical element 20 at the distal end of the stem 16. Light source 12 may comprise filters, as known to those skilled in the art, to reduce the damaging thermal effects of absorbed infrared radiation originating at the light source. The light source 12 filter(s) can be used to selectively illuminate a surgical field with different colors of light, such as to excite a surgical dye.

Fiber(s) 22 (and/or 14, depending on the embodiment) is/are terminated by optically coupling to optical element 20. Fiber 22 and optical element 20 can be optically coupled through direct contact, or through a variable intermediary air gap 24. The size of air gap 24 can be adjusted via adjusting means 40 of FIGURE 4, which can be used by, for example, a surgeon to adjust the linear displacement of fiber 22, as discussed herein. Optical element 20 is an optical grade sapphire diffuser having a hemispherical shape. Optical element 20 can comprise a polished flat surface 25 at the distal end of stem 16 (i.e., facing out towards a surgical field) and a hemispherical surface 26 facing the distal end of fiber 22. Optical element 20 is sized for housing within stem 16 (e.g., a 19 to 30 gauge cannula). For example, optical element 20 can have a diameter of about .75 mm to about .4 mm. The flat surface 25 of optical element 20 can be co-incident with the open aperture at the distal end of stem 16.

As shown in FIGURE 2, optical element 20 comprises a smooth hemispherical sapphire. Optical element 20 can be a commercially available sapphire element known to those familiar with the art. Non-parallel light rays 30 from fiber 22 strike the refractive spherical surface 26, resulting in a diffused (isotropic), wide-angle pattern of light 33 exiting optical element 20 at its flat surface 25. The non-coherent light rays 30 entering refractive spherical surface 26 are bent toward the principal optical axis, with the more peripheral rays bent at sharper angles to provide a wider angle of illumination. The optical element 20, optically coupled to the distal end of the light carrying fiber 22, is housed inside stem 16 (e.g., a small-gauge cannula of about 19 to 30 gauge). Stem 16 is itself operably coupled to the handpiece 10, which can be either a re-usable or a disposable handpiece 10.

FIGURE 3 illustrates the use of one embodiment of the variable-intensity, wide-angle illuminator of this invention in an ophthalmic surgery. In operation, handpiece 10 delivers a beam of incoherent light through stem 16 (via optical fiber 22 and/or fiber optic cable 14) and through optical element 20 to illuminate a retina 28 of an eye 30. The collimated light delivered through handpiece 10 to optical element 20 is generated by light source 12 and delivered to illuminate the retina 28 by means of fiber optic cable 14 and coupling system 32. Optical element 20 spreads the light beam delivered from light source 12 over as large an area of the retina as, for example, a microscopic wide-angle objective lens permits a surgeon to see. The embodiments of the wide-angle illuminator of this invention can provide illumination angles up to about 160 degrees.

FIGURE 4 provides another view of a wide-angle illuminator according to the teachings of this invention showing more clearly an embodiment of adjusting means 40. In this embodiment, adjusting means 40 comprises a slide button, as known to those skilled in the art. Activation of adjusting means 40 on handpiece 10 by, for example, a gentle and reversible sliding action, can cause the fiber 22 to move away from or towards optical element 20 by an amount determined and adjusted by sliding adjusting means 40. Adjusting the linear displacement of the optical fiber 22 within stem 16 in this way will change the distance between the optical element 20 and the optical fiber 22. When optical fiber 22 is closer to optical element 20, the non-parallel light rays 30 exiting optical fiber 22 will be diffracted less before entering optical element 20 than when optical fiber 22 is farther away from optical element 20.

The greater refraction of the non-parallel light rays 30 when optical element 20 and fiber 22 are farther apart will cause some of the non-parallel light rays 30 to enter the refractive spherical surface 26 at steeper angles. The result will be a greater degree of refraction within optical element 20, and, consequently, a pattern of light 32 exiting from optical element 20 at a greater angle of illumination. Thus, the angle of illumination and the amount of illumination provided by optical element 20 to illuminate the surgical field (e.g., the retina 28 of an eye 30) can easily be adjusted by a surgeon by adjusting the linear displacement of optical fiber 22 (i.e., adjusting the distance between optical fiber 22 and optical element 20). In this way, a surgeon can adjust the amount of light spread over a surgical field as desired to optimize the viewing field while minimizing glare. The adjusting means 40 of handpiece 10 can be any adjusting means known to those familiar with the art.

In one embodiment of the variable-intensity, wide-angle illuminator of the present invention, a simple mechanical locking mechanism, as known to those skilled in the art, can permit the illumination angle (distance between optical element 20 and fiber 22) to be fixed, until released and/or re-adjusted by the user via the adjusting means 40. Thus, the pattern of light 32 emanating from the distal end of stem 16 will illuminate an area over a solid angle *θ*, the angle *θ* being continuously adjustable by a user (e.g., a surgeon) via the adjusting means 40 of handpiece 10.

An advantage of the optical element 20 and of the embodiments of the variable-intensity, wide-angle illuminator of this invention, is that an operator can continuously vary the intensity and angle of illumination of the pattern of light 32 exiting optical element 20 to optimize viewing conditions within the surgical field. The pattern of light 32 from optical element 20 can thus be focused and controlled as desired by the operator. The embodiments of the variable-intensity, wide-angle illuminator of the present invention are therefore operable to adjust the angle and intensity of the light provided by light source 12 to substantially cover the area of the surgical field desired by a surgeon.

The embodiments of the variable-intensity, wide-angle illuminator of this invention provide several advantages over the prior art, such as maximizing light transmission by eliminating the requirement of multiple transmitting, reflecting, or diffracting optical elements, all of which can present sources of further transmission loss between a light source 12 and a target area to be illuminated. Further, the embodiments of this invention have an inherently high light flux capacity and a variable illumination angle, which will permit the operator to tailor the angular illumination requirements for a specific surgical environment. Additionally, a variable illumination angle allows an operator to adjust the intensity of the illumination using both source intensity variations and angle of incidence variations to minimize glare and shadowing in the surgical field. By varying the angle of illumination on a specific portion of the surgical field, an operator, such as a surgeon, can get an improved perception of spatial awareness.

A traditional fiber-optic illuminator with a polished face will produce an included illumination angle that is a function of the numerical aperture ("NA") of the fiber. NA defines the acceptance angle of entrance of the light from the light source into the fiber optic cable. Commonly, the fiber used for ophthalmic illumination applications has a typical NA of 0.5. This provides a calculated acceptance angle of 60 degrees in vacuo. Wide-angle viewing systems commonly used by ophthalmic surgeons typically have a viewing angle requirement greater than about 100 degrees in vivo. Thus, conventional fiber optic illuminators cannot provide a lighted field that matches the viewing system angle of visibility. The embodiments of the variable-intensity, wide-angle illuminator of this invention can provide an angle of illumination in excess of about 160 degrees (i.e., a range of illumination angles up to about 160 degrees).

Although the present invention has been described in detail herein with reference to the illustrated embodiments, it should be understood that the description is by way of example only and is not to be construed in a limiting sense. It is to be further understood, therefore, that numerous changes in the details of the embodiments of this invention and additional embodiments of this invention will be apparent to, and may be made by, persons of ordinary skill in the art having reference to this description. It is contemplated that all such changes and additional embodiments are within the scope of this invention as claimed below. Thus, while the present invention has been described in particular reference to the general area of ophthalmic surgery, the teachings contained herein apply equally wherever it is desirous to provide wide-angle and variable illumination, and where contact with a transparent fluid might normally interfere with the ability to obtain wide-angle illumination.

## Claims

1. A small-gauge, wide-angle illuminator, comprising:
a handpiece (10), optically coupled to receive a light beam from a light source (12);
an optical fiber (22) or fiber optic cable (14), operably coupled to the handpiece, wherein the fiber receives the light beam from the light source; an optical element (20), optically coupled to a distal end of the fiber, for receiving the light beam and scattering the light beam to illuminate a surgical field, and
a cannula (16), operably coupled to the handpiece, for housing and directing the optical fiber and the optical element,
**characterized in that** the optical element comprises a hemispherically shaped sapphire having a circular surface (25) co-incident with an open aperture of the cannula (16), and a hemispherical surface (26) facing the optical fiber; and
the optical fiber (22) is operably coupled to the handpiece (10) to enable linear displacement of the optical fiber within the cannula (16).

2. The small-gauge, wide-angle illuminator of Claim 1, wherein the optical element (20) is a 19, 20 or 25 gauge optical element.

3. The small-gauge, wide-angle illuminator of Claim 1, wherein the cannula (16) and the handpiece are fabricated from biocompatible materials.

4. The small-gauge, wide-angle illuminator of Claim 1, wherein the optical fiber (22) is optically coupled at the distal end to the optical element (20) and at another end to an optical cable (14), wherein the optical cable is operably coupled to the light source (12) to transmit the light beam to the optical fiber, and wherein the optical cable comprises a first optical connector (32) operably coupled to the light source (12) and a second optical connector (32) operably coupled to the handpiece (10).

5. The small- gauge, wide-angle illuminator of Claim 4, wherein the optical cable (14) gauge and the optical fiber (22) gauge are equal.

6. The small-gauge, wide-angle illuminator of Claim 4, wherein the optical cable (14) comprises a plurality of optical fibers.

7. The small-gauge, wide-angle illuminator of Claim 4, wherein the first and second optical connectors (32) are SMA optical fiber connectors.

8. The small-gauge, wide-angle illuminator of Claim 1, wherein the optical fiber (22) gauge and the optical element (20) gauge are equal.

9. The small-gauge, wide-angle illuminator of Claim 1, further comprising a means (40) for adjusting the linear displacement of the optical fiber (22).

10. The small-gauge, wide-angle illuminator of Claim 9, wherein the means (40) for adjusting comprises a push/pull mechanism.

11. The small-gauge, wide-angle illuminator of Claim 10, wherein adjusting the linear displacement causes the optical fiber (22) to move away from or towards the optical element (20) by an amount corresponding to the change in linear displacement.

12. The small- gauge, wide-angle illuminator of Claim 11, wherein the amount of linear displacement of the optical fiber (22) determines an angle of illumination and an amount of illumination (33) provided by the optical element (20) to illuminate the surgical field.

13. The small-gauge, wide-angle illuminator of any one of Claims 1 to 12, wherein the angle of illumination is between 20 and about 160 degrees.

14. The small-gauge, wide-angle illuminator of any one of Claims 1 to 13, wherein the light beam comprises a beam (30) of relatively incoherent light.

15. The small-gauge, wide-angle illuminator of any one of Claims 1 to 14, wherein the light source (12) is a xenon light source.

16. The small-gauge, wide-angle illuminator of any one of Chims 1 to 15, wherein the optical element (20) is about 2 millimeters long.

## Patentansprüche

1. Ein Weitwinkelilluminator mit geringem Durchmesser, umfassend:
ein Handstück (10), das zum Empfang eines Lichtstrahles von einer Lichtquelle (12) optisch gekoppelt ist;
eine optische Faser (22) oder ein faseroptisches Kabel (14), das betriebstechnisch mit dem Handstück gekoppelt ist, wobei die Faser den Lichtstrahl von der Lichtquelle empfängt;
ein optisches Element (22), das mit einem distalen Ende der Faser zum Empfang des Lichtstrahls und zum Streuen des Lichtstrahls zur Beleuchtung eines Operationsgebietes optisch gekoppelt ist; und
eine betriebstechnisch mit den Handstück gekoppelte Kanüle (16) zur Aufnahme und zur Führung der optischen Faser und des optischen Elements, **dadurch gekennzeichnet, daß**
das optische Element koinzident mit einer geöffneten Öffnung der Kanüle (16) einen halbkugelförmigen Saphir mit einer kreisförmigen Oberfläche (25) und einer halbkugelförmigen Oberfläche (26) umfaßt, die der optischen Faser zugewandt ist, und daß die optische Faser (22) betriebstechnisch mit dem Handstück (10) gekoppelt ist, um eine lineare Verschiebung der optischen Faser in der Kanüle (16) zuzulassen.

2. Weitwinkelilluminator mit geringem Durchmesser nach Anspruch 1, wobei das optische Element (20) ein optisches Element mit Durchmessern 19, 20 oder 25 ist.

3. Weitwinkelilluminator mit geringem Durchmesser nach Anspruch 1, wobei die Kanüle (16) und das Handstück aus bikompatiblen Materialen hergestellt sind.

4. Weitwinkelilluminator mit geringem Durchmesser nach Anspruch 1, wobei die optische Faser (22) am distalen Ende mit dem optischen Element (20) und an einem anderen Ende mit einem optischen Kabel (14) optisch gekoppelt ist, wobei das optische Kabel betriebstechnisch mit der Lichtquelle (12) zur Übertragung des Lichtstrahls zur optischen Faser gekoppelt ist und das optische Kabel einen ersten optischen Stecker (32), der betriebstechnisch mit der Lichtquelle (12) gekoppelt ist, und einen zweiten optischen Stecker (32) umfaßt, der betriebstechnisch mit dem Handstück (10) gekoppelt ist.

5. Weitwinkelilluminator mit geringem Durchmesser nach Anspruch 4, wobei der Durchmesser des optischen Kabels (14) und der Durchmesser der optischen Faser (22) gleich sind.

6. Weitwinkelilluminator mit geringem Durchmesser nach Anspruch 4, wobei das optische Kabel (14) mehrere optische Fasern umfaßt.

7. Weitwinkelilluminator mit geringem Durchmesser nach Anspruch 4, wobei der erste und der zweite optische Stecker (32) optische SMA-Faserstecker sind.

8. Weitwinkelilluminator mit geringem Durchmesser nach Anspruch 1, wobei der Durchmesser der optischen Faser (22) und der Durchmesser des optischen Elements (20) gleich sind.

9. Weitwinkelilluminator mit geringem Durchmesser nach Anspruch 1, der des weiteren ein Mittel (40) zur Einstellung der linearen Verschiebung der optischen Faser (22) umfaßt.

10. Weitwinkelilluminator mit geringem Durchmesser nach Anspruch 9, wobei das Mittel (40) zur Einstellung einen Druck-Zug-Mechanismus umfaßt.

11. Weitwinkelilluminator mit geringem Durchmesser nach Anspruch 10, wobei die Einstellung der linearen Verschiebung bewirkt, daß sich die optische Faser (22) vom optischen Element (20) um einen Betrag wegbewegt oder darauf zu bewegt, der der Änderung der linearen Verschiebung entspricht.

12. Weitwinkelilluminator mit geringem Durchmesser nach Anspruch 11, wobei der Betrag der linearen Verschiebung der optischen Faser (22) einen Beleuchtungswinkel und eine Beleuchtungsstärke (33) festlegt, der bzw. die vom optischen Element (20) zur Beleuchtung des Operationsgebietes bereitgestellt wird.

13. Weitwinkelilluminator mit geringem Durchmesser nach einem der Ansprüche 1 bis 12, wobei der Beleuchtungswinkel zwischen 20° und ungefähr 160° liegt.

14. Weitwinkelilluminator mit geringem Durchmesser nach einem der Ansprüche 1 bis 13, wobei der Lichtstrahl einen Strahl (30) aus relativ inkoherentem Licht umfaßt.

15. Weitwinkelilluminator mit geringem Durchmesser nach einem der Ansprüche 1 bis 14, wobei die Lichtquelle (12) eine Xenonlichtquelle ist.

16. Weitwinkelilluminator mit geringem Durchmesser nach einem der Ansprüche 1 bis 15, wobei das optische Element (20) ungefähr 2 Millimeter lang ist.

## Revendications

1. Dispositif d'éclairage à grand angle, de petit calibre, comportant :
une pièce à main (10), couplée optiquement pour recevoir un faisceau de lumière provenant d'une source de lumière (12),
une fibre optique (22) ou un câble à fibre optique (14) couplé de manière opérationnelle à la pièce à main, la fibre recevant le faisceau de lumière provenant de la source de lumière,
un élément optique (20), couplé optiquement à une extrémité distale de la fibre, pour recevoir le faisceau de lumière et diffuser le faisceau de lumière afin d'éclairer un champ chirurgical, et
une canule (16), couplée de manière opérationnelle à la pièce à main, pour recevoir et diriger la fibre optique et l'élément optique,
**caractérisé en ce que** l'élément optique comporte un saphir mis en forme de manière hémisphérique ayant une surface circulaire (25) qui coïncide avec une ouverture de la canule (16), et une surface hémisphérique (26) en vis-à-vis de la fibre optique, et la fibre optique (22) est couplée de manière opérationnelle à la pièce à main (10) pour permettre un déplacement de la fibre optique dans la canule (16).

2. Dispositif d'éclairage à grand angle, de petit calibre, selon la revendication 1, dans lequel l'élément optique (20) est un élément optique de calibre 19, 20 ou 25.

3. Dispositif d'éclairage à grand angle, de petit calibre, selon la revendication 1, dans lequel la canule (16) et la pièce main sont fabriquées en matériaux biocompatibles.

4. Dispositif d'éclairage à grand angle, de petit calibre, selon la revendication 1, dans lequel la fibre optique (22) est couplée optiquement, à l'extrémité distale, à l'élément optique (20), et au niveau de l'autre extrémité à un câble optique (14), dans lequel le câble optique est couplé de manière opérationnelle à la source de lumière (12) pour transmettre le faisceau de lumière à la fibre optique, et dans lequel le câble optique comporte un premier connecteur optique (32) couplé de manière opérationnelle à la source de lumière (12), et un second connecteur optique (32) couplé de manière opérationnelle à la pièce à main (10).

5. Dispositif d'éclairage à grand angle, de petit calibre, selon la revendication 4, dans lequel le calibre du câble optique (14) et le calibre de la fibre optique (22) sont égaux.

6. Dispositif d'éclairage à grand angle, de petit calibre, selon la revendication 4, dans lequel le câble optique (14) comporte une pluralité de fibres optiques.

7. Dispositif d'éclairage à grand angle, de petit calibre, selon la revendication 4, dans lequel les premier et second connecteurs optiques (32) sont des connecteurs à fibre optique de type SMA.

8. Dispositif d'éclairage à grand angle, de petit calibre, selon la revendication 1, dans lequel le calibre de la fibre optique (22) et le calibre de l'élément optique (20) sont égaux.

9. Dispositif d'éclairage à grand angle, de petit calibre, selon la revendication 1, comportant en outre des moyens (40) pour ajuster le déplacement linéaire de la fibre optique (22).

10. Dispositif d'éclairage à grand angle, de petit calibre, selon la revendication 9, dans lequel les moyens (40) pour ajuster comportent un mécanisme de poussée/traction.

11. Dispositif d'éclairage à grand angle, de petit calibre, selon la revendication 10, dans lequel un ajustement du déplacement linéaire amène la fibre optique (22) à s'éloigner de l'élément optique (20), ou à s'en rapprocher, d'une quantité correspondant au changement de déplacement linéaire.

12. Dispositif d'éclairage à grand angle, de petit calibre, selon la revendication 11, dans lequel la quantité de déplacement linéaire de la fibre optique (22) détermine un angle d'éclairage et une quantité d'éclairage (33) fournie par l'élément optique (20) pour éclairer le champ chirurgical.

13. Dispositif d'éclairage à grand angle, de petit calibre, selon l'une quelconque des revendications 1 à 12, dans lequel l'angle d'éclairage est compris entre environ 20 et environ 160 degrés.

14. Dispositif d'éclairage à grand angle, de petit calibre, selon l'une quelconque des revendications 1 à 13, dans lequel le faisceau de lumière comporte un faisceau (30) d'une lumière relativement incohérente.

15. Dispositif d'éclairage à grand angle, de petit calibre, selon l'une quelconque des revendications 1 à 14, dans lequel la source de lumière (12) est une source de lumière au xénon.

16. Dispositif d'éclairage à grand angle, de petit calibre, selon l'une quelconque des revendications 1 à 15, dans lequel l'élément optique (20) a une longueur d'environ 2 mm.
